# EUROPÄISCHE PATENTSCHRIFT

(11) **EP 1 529 774 B1**
(45) Veröffentlichungstag und Bekanntmachung des Hinweises auf die Patenterteilung: **18.02.2009**
(21) Anmeldenummer: 04025917.8
(22) Anmeldetag: 02.11.2004
(51) Int. Cl.: C07C 227/18, C07C 319/20, A23L 1/304

(54) **Verfahren zur Herstellung einer Organozinkverbindung und deren Verwendung in einer zinkhaltigen Zusammensetzung für die Nahrungsergänzung**
Process for the preparation of an organozinc derivative and its use in a zinc-containing composition as nutritional supplement
Procédé pour la préparation d'un dérivé organique du zinc et son utilisation dans une composition contenant du zinc utile comme complément alimentaire

(30) Priorität: 04.11.2003 DE 10352129
(43) Veröffentlichungstag der Anmeldung: 11.05.2005
(60) Teilanmeldung: 08020141.1
(73) Patentinhaber: Grillo Zinkoxid GmbH, 38644 Goslar (DE)
(72) Erfinder: JAHN, Dr. rer. nat. Dip.-Chem. Burkhardt, 38667 Bad Harzburg (DE); SCHUBERT, Peter, Dipl.-Chem., 38690 Vienenburg (DE)
(74) Vertreter: Läufer, Martina

(56) Entgegenhaltungen:
- EP-A- 0 514 553
- WO-A-00/53032
- WO-A-01/68087
- WO-A-02/30947
- WO-A-20/04021802
- DE-A- 2 436 946
- US-A- 4 579 962
- US-A- 4 830 716
- WILSON R.B. ET AL.: "Structural characterization of bis(L-methionato)zinc (II), Zn(L-met)2" INORGANIC CHEMISTRY., Bd. 16, Nr. 6, 1977, Seiten 1498-1502, XP008042395 USAMERICAN CHEMICAL SOCIETY. EASTON.
- AKIHAMA S. ET AL.: "Antiviral effect of zinc complexes on Japanese B Encephalitis virus" CHEMICAL AND PHARMACEUTICAL BULLETIN., Bd. 10, Nr. 12, 1962, Seiten 1254-1257, XP008042423 JP PHARMACEUTICAL SOCIETY OF JAPAN. TOKYO.

## Beschreibung

Die Erfindung betrifft ein Verfahren zur Herstellung einer Organozinkverbindung, insbesondere als Nahrungsergänzungsmittel, bei welchem eine anorganische Zinkverbindung mit einer organischen Säure, insbesondere einer Aminosäure, umgesetzt wird.

Seit langem ist bekannt, dass das Element Zink für die Entwicklung und Gesunderhaltung von Pflanzen, Tieren und Menschen wesentlich ist. Zink aktiviert verschiedene Enzyme und ist Bestandteil vieler wichtiger Metalloenzyme; es ist daher ein wesentliches intrazelluläres Spurenelement.

Besonders in der industriellen Massentierhaltung werden Spurenelemente und Mineralien als Futterzusätze für eine ausgewogene Ernährung und ein gutes Gedeihen der Tiere benötigt. Spezielle Futterzusammensetzungen, beispielsweise mit hohem Kalziumgehalt, können allerdings die Bioverfügbarkeit von Zink in der Nahrung beeinträchtigen. Ebenso kann die Zinkzufuhr bei Diäten von Mensch und Tier zu gering sein. Einzeln oder im Gemisch mit anderen Zusatzstoffen werden daher seit längerer Zeit Zinkverbindungen für die Nahrungsergänzung von Mensch und Tier gegeben.

Als Nahrungs- oder Futter-Ergänzungsmittel sind sowohl anorganische wie auch organische Zinkverbindungen im Gebrauch. Unter anderem werden Zinkoxid (ZnO), Zinksulfat (ZnSO₄, i.a. als Hydrat), Zinkchlorid (ZnCl₂) und organische Zinkverbindungen verwendet. Von organischen Salzen erhofft man sich im Allgemeinen eine höhere Bioverfügbarkeit. Speziell sind auch Aminosäure-Zink-Komplexe bekannt. Dies bringt den Vorteil mit sich, dass gemeinsam sowohl Zink als auch Aminosäure verabreicht werden können, da den Futterzusatzstoffen ohnehin vielfach Aminosäuren oder Aminosäuregemische beigefügt sind. In der Tierernährung verwendete Aminosäuren sind vor allem: Methionin, Histidin, Phenylalanin, Lysin, Thryptophan, Threonin, Isoleucin, Valin, Arginin und Leucin (essentielle Aminosäuren für Mensch, Geflügel, Schwein).

Für die Futtermittelergänzung sind unter anderem Zink-Aminosäure 1:1-Komplexe bekannt, wie beispielsweise Zinkmethionin- oder Zinklysin-Komplexe sowie Aminosäuregemisch-Zink-Verbindungen. Auch als Zinkgluconat wird Zink häufig gegeben, und zwar für Mensch und Tier.

Aus der DE 24 36 946 A1 ist die Herstellung von Zink-Methionin 1:1-Komplexsalzen der Form Zink(methionato-N,O)Xₙ bekannt, wobei X für ein beliebiges ein- oder zweiwertiges Anion steht (n = 1;2), vorzugsweise Chlorid, Hydrogensulfat oder Acetat.

Die Herstellung erfolgt, indem die gewünschte Aminosäure, hier Methionin, einer heißen Lösung eines anorganischen Zinksalzes zugegeben wird, oder indem ein Hydratsalz mit Methionin erwärmt wird.

Die Aufgabe der Erfindung bestand darin, für die Nahrungsergänzung alternative Organozinkverbindungen aus verfügbaren und preiswerten Ausgangsverbindungen herzustellen.

Diese Aufgabe wird gelöst durch ein Verfahren der eingangs genannten Art, bei dem als Zinkverbindung Zinkoxid eingesetzt wird, das Zinkoxid und die Säure gemischt werden, das Gemisch in Substanz, ohne Zugabe von Lösungsmittel durch Energiezufuhr mittels Wärme oder Bestrahlung auf eine Temperatur unterhalb der Zersetzungstemperatur der Säure, generell bis auf wenigsten 150 °C (gut unterhalb der Zersetzungstemperatur organischer Säuren), erwärmt und für die Reaktionszeit bei oder unterhalb dieser Temperatur gehalten wird.

Vorzugsweise werden organische Säuren verwendet, die bei Normaldruck bis zur Zersetzungstemperatur im festen Aggregatszustand vorliegen, so dass das Verfahren in fester Phase, d.h. im trockenen Gemisch, durchgeführt wird.

Ein Vorteil des Verfahrens liegt darin, dass Zinkoxid industriell in ausreichendem Maßstab und in geeigneter Reinheit zur Verfügung steht. Für verschiedene Anwendungszwecke ist hochreines Zinkoxid in pulverisierter Form erhältlich. Diese Zinkoxide erfüllen die Reinheitsanforderungen nationaler und internationaler Pharmakopeen wie USP, DAB 10 etc. und die der europäischen Futtermittelverordnung. Wegen mangelnder Löslichkeit des Zinkoxids in Wasser kann jedoch die Umsetzung von ZnO mit organischen Säuren zu Salzen dieser organischen Säuren oder zu Organometallkomplexsalzen nicht sinnvoll in Lösung durchgeführt werden.

Überraschenderweise wurde nun gefunden, dass bei geeigneter Temperaturführung Zinkoxid und organische Säuren in der Feststoffphase, also trocken umgesetzt werden können, ohne dass in einem für ein Nahrungsergänzungsmittel störenden Umfang Neben- oder Zersetzungsprodukte entstünden. Es konnte gezeigt werden, dass dabei ein Säure-Zink 2:1-Komplex oder ein zweiwertig (durch 2 einwertige oder 1 zweiwertiges Säureanion) organisch substituiertes Zinksalz erhalten wird.

Für die Aminosäure-Komplexe wird derzeit davon ausgegangen, dass sich ein "bis({*Name der Aminosäure*}ato-N,O)-Zink-Komplex bildet, oder allgemein ein binärer Komplex des zweifach positiv geladenen Zinkkations mit zwei Aminosäureanionen, vermutlich über den Sauerstoff der Carboxylatgruppen und den Stickstoff der Aminogruppen gebunden Diese Form des 2:1-Komplexes ist für Methionin belegt (CAS Registrierungsnummer 151214-86-7 für Zink,bis(Dmethioninato-N,O)-, (T-4)- (9Cl)). Bei Einsatz optisch reiner Aminosäuren werden auch die entsprechenden Komplexe gefunden. Da als Futtermittelzusatz üblicherweise DL-Methionin gegeben wird, erhielte man einen bis(DLmethioninato-N,O)-Zink-Komplex. Es ist nicht ausgeschlossen, dass sich in der Feststoffphase auch andere Mischformen bilden könnten, z. B. ein Methioninato (methioninato-N,O,S)-Zink-Komplex, bei dem das erste Methionin nur einfach über die Carboxylatgruppe koordiniert ist. Auch polymere Komplexe sind möglich. (Zur Struktur von Aminosäure-Zink-Komplexen, siehe: M. Rombach et al. "Coordination modes of aminoacids to Zinc", Inorganica Chimica Acta, 334 (2002) 25-33).

Die Komplexierung als solche wurde durch die Veränderung der Carbonylschwingung der Carboxylgruppe (²³_{coo}⁻) in dem erfindungsgemäß erhaltenen Verfahrensprodukt nachgewiesen.

Für andere 2:1-Verbindungen aus organischer Säure und Zink, wie sie nach dem erfindungsgemäßen Verfahren erhalten werden, sind verschiedene Strukturen möglich. Es kann sich um Carbonsäure-Salze handeln, bei denen je ein Zinkkation mit zwei Carboxylatanionen stöchiometrisch gebunden oder substituiert ist. Die Bindung kann über eine ionische oder vorwiegend ionische Wechselwirkung oder ebenfalls chelatisiert erfolgen, wobei die CarboxylatGruppe als bidentale Gruppe symmetrisch über beide Sauerstoffatome mit dem Zink koordiniert sein kann, wie im Zinkacetat (Zn(OOC-CH₃)₂) 2H₂O. Auch polymere Brückenstrukturen sind möglich.

Generell könnte die Reaktion mit ZnO mit folgender Summenformel beschrieben werden:
2 YH + ZnO ZnY₂ + H₂O
(Y = (Carbon-)Säureanion)

Es können eine oder mehrere organische Säuren im Gemisch verwendet werden. Vorzugsweise wird wenigstens eine der folgenden Säuren eingesetzt: Methionin, Glycin, Lysin, Leucin, Cystein, Phenylalanin, Histidin, Valin, Prolin, Tryptophan, Isoleucin, Arginin, Asparagin, Thyrosin, Threonin, Weinsäure, Citronensäure, Oxalsäure, Succinsäure, Adipinsäure, Gluconsäure, Alanin, Serin, Cystein, Glutamin, Glutaminsäure, Asparaginsäure, Malonsäure, Maleinsäure, Fumarsäure, jegliche Aminosäuren aus durch Hydrolyse aufgespaltenem Sojaprotein.

Für die Verwendung als Nahrungs- oder Futtermittelzusatz ist es als besonders vorteilhaft anzusehen, dass solche Säuren verwendet werden, die dem Futter/der Nahrung ohnehin zugesetzt werden sollen, also insbesondere Aminosäuren, einzeln oder im Gemisch, wobei Methionin und/oder Lysin besonders bevorzugt sind.

Das Zinkoxid wird als feinkristalline Substanz mit spezifischen Oberflächen von 1 bis 100 m²/gr eingesetzt wird, bevorzugt ZnO-Pulver mit einer BET-Oberfläche von 4 bis 10 m²/gr eingesetzt.

Das Verfahren wird allgemein so durchgeführt, dass das pulverförmige Zinkoxid und die ebenfalls vorzugsweise pulverförmige organische Säure zunächst gemischt werden. Die Reaktion wird erfindungsgemäß in Substanz, d.h. ohne Zusatz von Lösungsmittel durchgeführt. Andere Zusatzstoffe, die physikalisch in das Nahrungs- bzw. Futter-Ergänzungsmittel mit eingebunden werden sollen, können dem Gemisch zusätzlich beigegeben werden, soweit sie in dem für die Reaktion erforderlichen Temperaturbereich stabil sind. Dem Gemisch aus allen Edukten wird nun Energie zugeführt, um die Reaktion zwischen Zink und Säure auszulösen. Vor der Energiezufuhr kann das Gemisch zusätzlich durch Rütteln oder entsprechende Maßnahmen verdichtet werden. Die Energiezufuhr kann durch Wärme oder Bestrahlung erfolgen, dabei wird das Reaktionsgut vorzugsweise in Bewegung gehalten.

Die Bestrahlung erfolgt vorzugsweise mit Mikrowellen. Die Anwendung von Infrarot erscheint möglich, ist jedoch derzeit nicht bevorzugt.

Alternativ kann der Eduktansatz in einem konventionellen Wärmetauscher erwärmt werden, oder das trockene Gemisch kann in einem Drehrohrofen behandelt werden. Dem Fachmann sind Möglichkeiten zur schonenden Erwärmung trockener Gemische hinlänglich bekannt.

Das Verfahren wird so geführt, dass die Energie temperaturgesteuert zugeführt wird. In einer ersten Aufwärmphase wird vorzugsweise konstant(ΔE/t = const.) Energie zugeführt. Es wird jedoch bis maximal 5 °C, vorzugsweise nur bis maximal 10 °C unter die Zersetzungstemperatur der Säure erwärmt, d.h. allgemein nur bis zu einer solchen Temperatur, dass eine Zersetzung der Säure in nennenswertem Ausmaß ausgeschlossen werden kann. Vorzugsweise erfolgt eine Erwärmung auf wenigstens 150 °C, weiter vorzugsweise wenigstens 170 °C, im nachfolgenden Ausführungsbeispiel um 175 °C. Die Temperatur wird dann über einen bestimmten Zeitraum bei gedrosselter Energiezufuhr oder ggf. auch unter Kühlung oder zeitweiser Kühlung konstant gehalten. Auf eine möglichst gleichmäßige Erwärmung über den gesamten Ansatz, d.h. das gesamte Reaktionsvolumen, ist zu achten.

Nach der bemessenen Reaktionszeit wird das Reaktionsgemisch ohne Zufuhr weiterer Energie oder unter Kühlung abkühlen gelassen. Der Fachmann wird das Verhältnis von Maximaltemperatur zu Reaktionsdauer unter anderem unter Berücksichtigung des gewünschten Umsatzes bestimmen. Hierzu können einfache Vorversuche durchgeführt werden. Die Reaktionszeit kann sinnvollerweise zwischen 15 Minuten und 2 Stunden liegen. Die Reaktion könnte auch kontinuierlich in einem Durchlaufofen gefahren werden.

Versuche haben ergeben, dass ein Umsatz von 95 bis 97 % bezogen auf den Säureverbrauch innerhalb praktikabler Reaktionszeiten von nicht mehr als einigen Stunden möglich ist.

Ein erheblicher Vorteil des Verfahrens liegt darin, dass direkt, ohne dass weitere Verfahrensschritte wie Kristallisation, Sprühtrocknung, Filtration erforderlich wären, ein unmittelbar als Futtermittel-Zusatzstoff einsetzbares Produkt entsteht. Dieses Produkt ist aus industriell verfügbaren, relativ preiswerten Ausgangsstoffen erhältlich.

Das Zinkoxid kann innerhalb des erfindungsgemäßen Verfahrens im stöchiometrischen Verhältnis zur Säure, aber auch bewusst im Überschuss eingesetzt werden.

Da Zinkoxid selbst in großem Umfang als Futtermittelzusatzstoff verwendet wird, ergibt sich zumindest bei Einsatz des Zinkoxids im Überschuss immer ein sinnvolles Futterergänzungs- oder -zusatzmittel. Zinkoxid wird außer als Spurenelement in der Tierernährung auch zur Darmpflege gegeben. Eine Kombination aus ZnO mit einem organisch gebundenen Zinksalz bzw. -chelat wird daher in vielen Fällen zweckmäßig und wünschenswert sein. Die unterschiedlichen Freisetzungswege und Freisetzungskinetiken von ZnO und ZnX₂, wobei X für die im Rahmen dieser Erfindung verwendeten organischen Säuren steht, erleichtern die optimale Verteilung des Zinks im Körper, so dass sich hieraus ein weiterer Zusatznutzen ergibt.

Wird das Zinkoxid im Rahmen der Erfindung mit Aminosäuren umgesetzt, ist praktisch jedes Mischungsverhältnis von ZnO und Aminosäure möglich. Erhalten wird ein Gemisch aus restlichem bzw. überschüssigem Zinkoxid, dem gebildeten Chelat und restlicher bzw. überschüssiger Aminosäure. Die Verfahrensbedingungen können so eingestellt werden, dass ein für den jeweiligen Anwendungszweck optimales Produkt erhalten wird.

Die Reaktion zwischen der organischen Säure und dem Zinkoxid findet an der Oberfläche der Zinkoxid-Teilchen statt. Aufgrund der besonderen Verfahrensführung bei der Erfindung entsteht daher die erfindungsgemäße Organozinkverbindung, d.h. das erfindungsgemäße Chelat (sofern nicht im Einzelfall eine eher als Salz zu bezeichnende Verbindung vorliegt), physikalisch assoziiert mit einem Zinkoxid-Teilchen, bzw. physikalisch in dieses eingebunden oder daran anhaftend. Für den Fall, dass die Umsetzung nicht stöchiometrisch und praktisch vollständig erfolgt, enthält demnach die zinkhaltige Zusammensetzung das ZnO und die organisch koordinierte oder substituierte Zinkverbindung wenigstens teilweise physikalisch miteinander assoziiert. Gegenüber einer Mischung aus Zinkoxid und einem anderen Zinksalz ergibt sich der zusätzliche Vorteil, dass sich dieses Produkt weniger oder nicht entmischen wird.

Vorzugsweise enthält die für die Nahrungsergänzung von Mensch und Tier verwendete zinkhaltige Zusammensetzung neben Zinkoxid eine der folgenden Komplexverbindungen: binäres Zinkmethionat (Zn(Met)₂), insbesondere bis(DLmethioninato-N,O)-Zink oder bis(L-methioninato-N,O)-Zink, binäres Zinklysinat (Zn(Lys)₂), insbesondere bis(DL-lysinato-N,O)-Zink oder bis(L-lysinato-N,O)-Zink, Zinkmethionatcysteinat (Zn(Met)(Cys)), insbesondere (DL-methioninato-N,O)-DLcysteinato-N,O-Zink.

Im Folgenden wird die Erfindung anhand von Beispielen und analytischen Untersuchungen näher dargestellt. Die Beispiele dienen allein illustrativen Zwecken, ohne Beschränkung der Allgemeingültigkeit der oben dargestellten Erfindungsprinzipien.

### BEISPIELE

### 1. Allgemeine Daten zu Geräten und Reaktionsansätzen

Die Mikrowellen-gestützten Synthesen wurden durchgeführt in einem PCgesteuerten Labormikrowellensystem "ETHOS 1600" der Firma MLS (Leutkirch) mit 1600 Watt maximaler Leistung und 2,45 GHz Mikrowellenfrequenz.

Die im Folgenden genannten Reaktionsdaten beziehen sich auf 2,0 g- bzw. 4,5 g-Ansätze von 1:1 Mischungen (Molverhältnis) der Reaktanden Zinkoxid und Säure. Diese wurden in zylindrischen Reaktionsgefäßen mit einem Innendurchmesser von 10 mm umgesetzt. Der unterstöchiometrische Anteil an ZnO wurde in Abstimmung auf den mittleren Teilchendurchmesser des eingesetzten Zinkoxids gewählt, der ca. 1 µm betrug (mittlerer Teilchendurchmesser bestimmt durch Lichtstreuungsverfahren, entsprechend einer spezifischen Oberfläche (BET-Oberfläche) von ca. 4m²/gr), da die Umsetzbarkeit des Zinkoxids grundsätzlich beschränkt ist, weil die Reaktion nur an der Oberfläche der Zinkoxidteilchen stattfindet. Nicht umgesetztes Zinkoxid zeigt sich in den IR-Spektren der Produkte mit einer Bande bei etwa 440 cm⁻¹.

### 2. Experimentelle Ergebnisse

### 2.1. Vorversuche

Für einen Ansatz aus Zinkoxid und Methionin wurden Vorversuche zur Zersetzung des Methionins unter Mikrowellenbedingungen durchgeführt. Oberhalb von etwa 185 °C (gemessen mit zentral in der Reaktionsmischung angeordnetem Messfühler) trat merkliche Zersetzung des organischen Materials ein. Die Reaktionsmischung verfärbte sich dabei beige bis schwach bräunlich. Es traten flüchtige schwefelhaltige Anteile auf. Die Reaktionstemperatur wurde für die weiteren Versuchsreihen auf maximal 175 °C begrenzt.

Nasschemische Gewinnung von Methionin-Zink 1:1-Komplexen (Referenz)

68,0 g Zinkchlorid werden in 68,0 g Wasser aufgelöst, wobei die Lösung auf 90 °C erhitzt wird. Dann werden 74,6 g Methionin zugegeben, worauf die Temperatur 1 h auf 90 °C gehalten wird, um eine Zinkmethioninchlorid Lösung herzustellen (Vorschrift nach DE 24 36 946 A1). Das Produkt kann einem Sprühtrockner zugeführt werden, um unter Gewinnung eines 1:1 Zink-Methioninkomplexes sprühgetrocknet zu werden (nach DE 38 12 653).

Nasschemische Gewinnung von Methionin-Zink 2:1-Komplexen (Referenz)

0,298 g Methionin und 0,136 g Zinkchlorid werden in 50 ml warmem Wasser gelöst, von einer geringen Trübung abfiltriert und tropfenweise mit n/5 Kalilauge versetzt, bis ein pH-Wert von 5,4 erreicht ist. Der schwerlösliche Methionin-Zink-Komplex fällt sofort aus.

Die Komplexbildung kann folgendermaßen beschrieben werden: 2 CH₃SCH₂CH₂CH(NH₂)COOH + Zn²⁺ + 2 X→ [CH₃SCH₂CH₂CH(NH₂)COO]₂Zn + 2 HX

Bei dem nasschemischen Verfahren muss die entstehende starke Säure abgefangen werden.

### 2.2. Versuchsreihen zu trockener Erwärmung

In zwei Versuchen mit der nachfolgend wiedergegebenen Reaktionsführung wurde reproduzierbar ein nahezu 100 %iger Umsatz bezogen auf den Methionin-Verbrauch erreicht:

| Mikrowellensynthese | |
|---|---|
| Mikrowellenleistung | 650 Watt |
| Aufheizperiode | Raumtemperatur bis 175 °C innerhalb von 10 min; |
| isotherme Phase | 30 min bei 175 °C; |
| Abkühlphase | innerhalb von etwa 15 min von 175 °C auf Raumtemperatur |
| Ausbeute | 70 bis 95 % Methionin-Umsatz |

Das Gemisch wurde nach Eintauchen des Temperaturfühlers, vor der Reaktion mit einem elektrischen Vibrationserzeuger verdichtet.

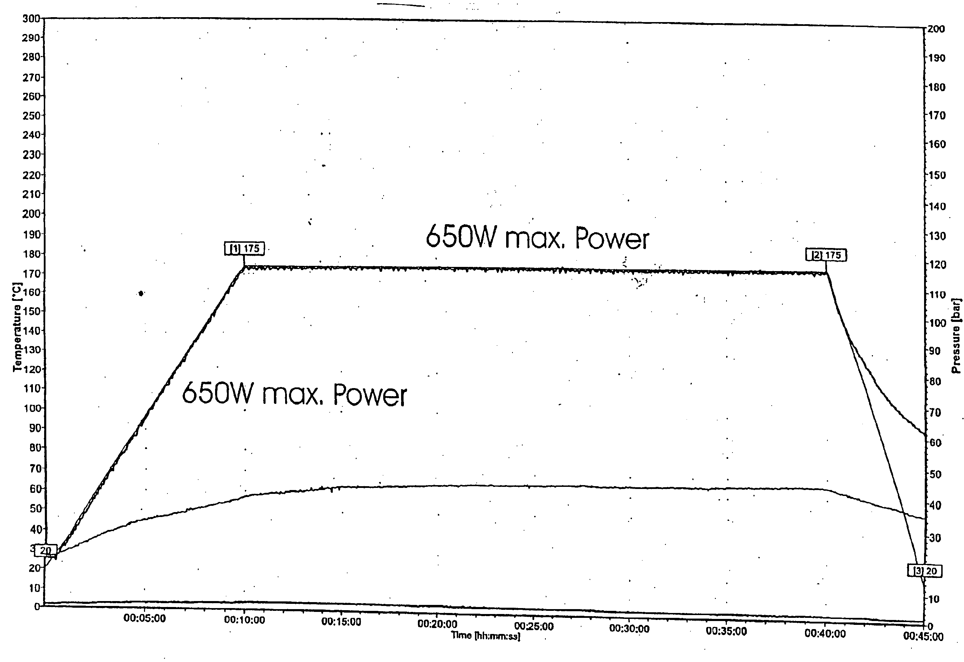

Das Geräteprotokoll mit den IST- und SOLL-Werten der Temperatur der Reaktionsmischung sowie dem Druckverlauf ist in Abb. 1 gezeigt. Die untere Kurve gibt den Druckverlauf wieder, die glatte obere Kurve gibt die Temperatur-SOLL-Werte an, die zweite obere Kurve die Temperatur-IST-Werte.

### 2.3. Analytik

Der analytische Nachweis des gebildeten Zink-Methionins gelingt unter anderem mittels IR-Spektroskopie. Die Carbonylschwingung der Carboxylgruppe (ν^{as}_{coo}-) des Produktes zeigt bei einer relativ geringfügigen Lageänderung von Δ =25cm⁻¹ eine signifikante Änderung der Signalform gegenüber dem Ausgangsmaterial Methionin: Während das Edukt eine starke Dreifachbande mit Schwerpunkt bei 1581 cm⁻¹ zeigt, ergibt das Produkt Zink-Methionin eine sehr starke Einfachbande bei 1606,6 cm⁻¹ mit leichter Rechtsschulter.

Auch die symmetrische Carboxylat-Streckschwingung hat in Edukt und Produkt eine unterschiedliche Lage und ist daher analytisch nutzbar: Während diese in Methionin bei 1413,7 cm⁻¹ erscheint, liegt sie im Produkt bei 1425,3 cm⁻¹. Vergleichbare Wellenzahlendifferenzen zwischen L-α-Methionin und Zinkkomplexen des Methionins sind bekannt (J. Liu et al., J. Therm. Anal. Cal. 1999, 58, 323).

Die ¹³C-NMR-spektroskopische Untersuchung (mit NOE-Verstärkung durchgeführt) zeigt eine deutlichere Signaltrennung. Dies ermöglicht auch die Anteilsbestimmung Produkt/Edukt über die Signalfläche. Das Carboxylkohlenstoff-Signal des Eduktes findet sich bei 177,2 ppm und damit etwa 11 ppm abgeschirmt ("rechts") vom Produktsignal bei 188.8 ppm (Werte bezogen auf den internen Standard TMSP-d₄ = 0,0 ppm).

Eine schnell nutzbare Aussage über den Reaktionsfortschritt ergibt sich außerdem aus dem Vorhandensein eines starken endothermen Peaks bei 350 °C in der thermischen Analyse mittels DSC (Differential Scanning Calorimetry). Während dieser auf das Produkt hinweist, gibt sich restliches Methionin durch ein nur schwach ausgeprägtes endothermes Signal nahe 280°C zu erkennen. Eine Abschätzung des Methionin-Verbrauchs ist mittels einer Kalibrierreihe für das 280°C-Signal möglich.

## Patentansprüche

1. Verfahren zur Herstellung einer Organozinkverbindung, insbesondere als Nahrungsergänzungsmittel, bei welchem eine anorganische Zinkverbindung mit einer organischen Säure, insbesondere einer Aminosäure, umgesetzt wird,
**dadurch gekennzeichnet,**
**dass** als Zinkverbindung Zinkoxid eingesetzt wird, das Zinkoxid und die Säure gemischt werden, das Gemisch in Substanz, ohne Zugabe von Lösungsmittel durch Energiezufuhr mittels Wärme oder Bestrahlung auf eine Temperatur unterhalb der Zersetzungstemperatur der Säure erwärmt und für die Reaktionszeit bei oder unterhalb dieser Temperatur gehalten wird.

2. Verfahren nach Anspruch 1, **dadurch gekennzeichnet, dass** organische Säuren verwendet werden, die bei Normaldruck bis zur Zersetzungstemperatur im festen Aggregatszustand vorliegen und das Verfahren in fester Phase durchgeführt wird.

3. Verfahren nach Anspruch 1 oder 2, **dadurch gekennzeichnet, dass** mehrere organische Säuren, vorzugsweise Carbonsäuren und insbesondere Aminosäuren im Gemisch verwendet werden.

4. Verfahren nach einem der Ansprüche 1 bis 3, **dadurch gekennzeichnet, dass** wenigstens eine der folgenden Säuren verwendet wird: Methionin, Glycin, Lysin, Leucin, Phenylalanin, Histidin, Valin, Prolin, Tryptophan, Isoleucin, Arginin, Asparagin, Thyrosin, Threonin, Weinsäure, Citronensäure, Oxalsäure, Succinsäure, Adipinsäure, Gluconsäure, Alanin, Serin, Cystein, Glutamin, Glutaminsäure, Asparaginsäure, Malonsäure, Maleinsäure, Fumarsäure, jegliche Aminosäuren aus durch Hydrolyse aufgespaltenem Sojaprotein.

5. Verfahren nach einem der Ansprüche 1 bis 4, **dadurch gekennzeichnet, dass** das Zinkoxid pulverförmig als feinkristalline Substanz mit spezifischen Oberflächen von 1 bis 100 m²/gr eingesetzt wird, bevorzugt ZnO-Pulver mit einer BET-Oberfläche von 4 bis 10 m²/gr.

6. Verfahren nach einem der Ansprüche 1 bis 5, **dadurch gekennzeichnet, dass** das Gemisch während der Energiezufuhr in Bewegung gehalten wird.

7. Verfahren nach einem der Ansprüche 1. bis 6, **dadurch gekennzeichnet, dass** die Bestrahlung mit Mikrowellen erfolgt.

8. Verfahren nach einem der Ansprüche 1 bis 7, **dadurch gekennzeichnet, dass** das Erwärmen in einem Drehrohrofen erfolgt.

9. Verfahren nach einem der Ansprüche 1 bis 8, **dadurch gekennzeichnet, dass** die Erwärmung bis maximal 5 °C, vorzugsweise maximal 10 °C unter die Zersetzungstemperatur der Säure geführt wird.

10. Verfahren nach einem der Ansprüche 1 bis 9, **dadurch gekennzeichnet, dass** die Reaktionszeit zwischen 15 Minuten und 2 Stunden gehalten wird.

11. Verfahren nach einem der Ansprüche 1 bis 10, **dadurch gekennzeichnet, dass** das Zinkoxid (ZnO) im Überschuss eingesetzt wird.

## Claims

1. A method of manufacturing an organic zinc compound, particularly as a nutritional supplement, in which an inorganic zinc compound is converted using an organic acid, particularly an amino acid, **characterised in that**
zinc oxide is used as the zinc compound, the zinc oxide and the acid are mixed together, the mixture is heated in substance, without the addition of a solvent, to a temperature below the decomposition temperature of the acid through the supply of energy by means of heat or radiation and is maintained at or below this temperature for the reaction time.

2. The method according to claim 1, **characterised in that** organic acids are used, which are present in the solid aggregate state under normal pressure up to the decomposition temperature and the process is conducted in the solid phase.

3. The method according to claim 1 or 2, **characterised in that** several organic acids, preferably carbonic acids and particularly amino acids are used in the mixture.

4. The method according to one of the claims 1 to 3, **characterised in that** at least one of the following acids is used: methionine, glycine, lysine, leucine, phenylalanine, histidine, valine, proline, tryptophane, isoleucine, arginine, asparagine, thyrosine, threonine, tartaric acid, citric acid, oxalic acid, succinic acid, adipinic acid, gluconic acid, alanine, serine, cysteine, glutamine, glutaminic acid, asparaginic acid, malonic acid, maleic acid, fumaric acid, any amino acids from soya protein split by hydrolysis.

5. The method according to one of the claims 1 to 4, **characterised in that** the zinc oxide is used in powder form as a fine, crystalline substance with specific surfaces of between 1 and 100 m²/g, preferably ZnO powder with a BET surface of between 4 and 10 m²/g.

6. The method according to one of the claims 1 to 5, **characterised in that** the mixture is agitated while energy is supplied.

7. The method according to one of the claims 1 to 6, **characterised in that** the radiation results from microwaves.

8. The method according to one of the claims 1 to 7, **characterised in that** the warming takes place in a rotary furnace.

9. The method according to one of the claims 1 to 8, **characterised in that** the warming is carried out up to a maximum of 5 °C, preferably a maximum of 10 °C, under the acid's decomposition temperature.

10. The method according to one of the claims 1 to 9, **characterised in that** the reaction time is held for between 15 minutes and 2 hours.

11. The method according to one of the claims 1 to 10, **characterised in that** the zinc oxide (ZnO) is used in excess.

## Revendications

1. Procédé pour la préparation d'un composé organique du zinc, en particulier comme complément alimentaire, dans lequel un composé de zinc anorganique est transformé avec un acide organique, en particulier un acide aminé, **caractérisé en ce que** le composé de zinc utilisé est un oxyde de zinc, ledit oxyde de zinc et l'acide sont mélangés, le mélange est chauffé en substance, sans addition de solvant, par un apport d'énergie par chaleur ou par irradiation, à une température inférieure à la température de décomposition de l'acide et est maintenu à cette température ou au-dessous de celle-ci pendant le temps de réaction.

2. Procédé selon la revendication 1, **caractérisé en ce que** l'on utilise des acides organiques, qui, sous pression normale, sont disponibles à l'état d'agrégat solide jusqu'à la température de décomposition, et le procédé est mis en oeuvre dans la phase solide.

3. Procédé selon la revendication 1 ou 2, **caractérisé en ce que** l'on utilise un mélange de plusieurs acides organiques, de préférence des acides carboxyliques et, en particulier, des acides aminés.

4. Procédé selon l'une quelconque des revendications 1 à 3, **caractérisé en ce que** l'on utilise au moins l'un des acides suivants : méthionine, glycine, lysine, leucine, phénylalanine, histidine, valine, proline, tryptophane, isoleucine, arginine, asparagine, thyrosine, thréonine, acide tartrique, acide citrique, acide oxalique, acide succinique, acide adipique, acide gluconique, alanine, sérine; cystéine, glutamine, acide de glutamine, acide d'asparagine, acide malonique, acide maléique, acide fumarique, tout acide aminé issu de la protéine du soja dédoublée par hydrolyse.

5. Procédé selon l'une quelconque des revendications 1 à 4, **caractérisé en ce que** l'oxyde de zinc est utilisé sous forme de poudre en tant que substance en fins cristaux avec des surfaces spécifiques de 1 à 100 m²/g, de préférence de la poudre ZnO avec une surface BET de 4 à 10 m²/g.

6. Procédé selon l'une quelconque des revendications 1 à 5, **caractérisé en ce que** le mélange est maintenu en mouvement pendant l'apport d'énergie.

7. Procédé selon l'une quelconque des revendications 1 à 6, **caractérisé en ce que** l'irradiation est effectuée avec des micro-ondes.

8. Procédé selon l'une quelconque des revendications 1 à 7, **caractérisé en ce que** le chauffage est effectué dans un four tubulaire rotatif.

9. Procédé selon l'une quelconque des revendications 1 à 8, **caractérisé en ce que** le chauffage est effectué jusqu'à 5°C maximum, de préférence 10°C maximum en dessous de la température de décomposition de l'acide.

10. Procédé selon l'une quelconque des revendications 1 à 9, **caractérisé en ce que** le temps de réaction est maintenu entre 15 minutes et 2 heures.

11. Procédé selon l'une quelconque des revendications 1 à 10, **caractérisé en ce que** l'oxyde de zinc (ZnO) est utilisé en excédent.
